(19) Europäisches Patentamt
European Patent Office
Office européen des brevets

(11) Publication number: 0 144 812
A1

# (12) EUROPEAN PATENT APPLICATION

(21) Application number: 84113636.9

(22) Date of filing: 12.11.84

(51) Int. Cl.⁴: C 07 D 307/32
A 61 K 31/365

(30) Priority: 22.11.83 IT 2380683

(43) Date of publication of application:
19.06.85 Bulletin 85/25

(84) Designated Contracting States:
AT BE CH DE FR GB IT LI LU NL SE

(71) Applicant: LABORATORIO FARMACEUTICO CT
Via Dante Alighieri, 71
I-18038 Sanremo(IT)

(72) Inventor: Tessitore, Pietro Tommaso
Via Dante Alighieri, 71
I-18038 Sanremo(IT)

(74) Representative: Melocchi, Paola
Via Ulisse Salis 28
I-20161 Milano(IT)

(54) **New derivatives of gamma-butyrolactone, method for the preparation thereof and pharmaceutical compositions containing them.**

(57) The present invention relates to new derivatives of γ-butyrolactone, in particular α-alkylamino-γ-butyrolactones, and pharmaceutically acceptable salts thereof, able to inhibit the alcohol and intoxicating drinks ingestion.

Furthermore, the invention relates to the method for the preparation of the new derivatives of γ-butyrolactone and to the pharmaceutical compositions containing them.

EP 0 144 812 A1

The present invention relates to new γ- butyrolactone deri= vatives, to the preparation method thereof and to the pharmaceu= tical compositions containing them.

In particular, the invention relates to α-alkylamino-γ-bu= tyrolactones and to the pharmaceutically acceptable salts there= of, able to inhibit drinking of alcohol and intoxicating drinks.

The derivatives of γ-butyrolactone, which are one object of the present invention are comprised in the following general for= mula (I):

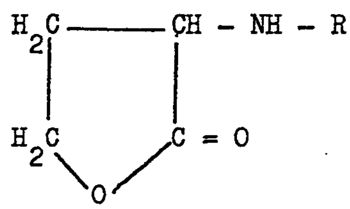

(I)

wherein R is a linear or branhed alkyl radical containing from 3 to 5 Carbon atoms.

It is known that an alcohol dependent subject, after he has ceased to ingest alcohol or intoxicating drinks, does not reach a complete detoxication by a "few days sobriety".
In fact it is deemed that several months of abstention from using alcohol are necessary before leading vital organs again to their rule. During the period following the suspension of ingesting alcohol, the therapy based on the use of drugs is often conside= red useful to discourage a patient from drinking again intoxica= ting drinks.

The more generally used drug in the abstinence period is di sulfiram, which seems to act as a chelating agent able to chelate Zn of the alcohol dehydrogenase, Fe and Mo of the aldehydo-dehy= drogenase, as well as Cu of the dopamine-β-hydroxylase.

However, disulfiram shows serious disadvantages when it is administered in order to make a patient free from alcohol-depen=

dence.

In fact, because of its interaction with ethanol, within a few minutes after having ingested very low amounts of alcohol, pa= tients treated with disulfiram show very particular side-effects. Patient begins to have a sensation of warmth in the face; skin, and particularly in the upper part of the chest and face becomes red, and he has a sensation of something lying heavy in his head and chest. Furthermore, this reaction can be accompanied also by difficulty in breathing, nausea, vomit, sudation, debility, diffi= cult sight and confusion.

Hitherto, the duration of the effect caused by a single dose of disulfiram is unknown; generally, it is thought that the effect ends within 12-24 hours. High levels of alcohol associated with the administration of high levels of disulfiram can cause a very serious reaction, even able to risk the life of the patient. The reaction is generally in proportion to the quantity of alcohol ingested and to the disulfiram dose.

Furthermore, disulfiram has in general a short effectiveness. For instance, a single dose of disulfiram may act only for 12-24 hours.

$\gamma$-Butyrolactones are compounds known from the literature. However, according to what described in the technical literature (William E. Klunk et al. Science, 217 , 1040 (1982)) they have the remarkable disadvantage to be convulsant at doses depending on the treated subject and therefore, although they show a certain effect in making the patient undependent on alcohol, they cannot be used.

$\beta$-Derivatives of $\gamma$-butyrolactone are also convulsant.

It has been now surprisingly found, and this is an object of the present invention, that $\alpha$-alkylamino-$\gamma$-butyrolactones compri sed in the general formula (I), and particularly $\alpha$-n.butylamino-

γ-butyrolactone ( which hereinafter will be indicated as PTT-1 for sake of brevity), are efficacious for treating alcohol dependence, and show some important advantages when compared with disulfiram, in that their mechanism of action is wholly different. In parti= cular:

(a) do not cause dangerous effects deriving from the association of the drug with alcohols, in particular with ethanol;

(b) their effectiveness lasts a long time;

(c) they are not convulsant.

The compounds comprised in the general formula (I), and in particular compound PTT-1, may be suitably administered by oral route or by i.m. or i.v. injection, according to the treated sub= ject; the administration by oral route is more suitable for humans, is more acceptable and therefore is preferred.

Usually, the best level of PTT-1 for humans is about 1 g/day, preferably divided in two administrations.
After 3 days of treatment with PTT-1 the suppression of the alco= hol desire is reached and lasts for about 30 days.

Another object of the present invention is the process for the preparation of compounds of the general formula (I). The pre paration can be performed according two methods:
Method A): the alkylhalide is allowed to react with the α-amino-γ-butyrolactone, at low temperature and in the presence of a ter tiary organic base, such as for instance pyridine.
Method B): an α-halo-γ-butyrolactone, preferably an α-chloro-γ-butyrolactone or α-bromo-γ-butyrolactone is allowed to react with the selected alkylamine, under the same conditions described in Method A).

α-Amino-γ-butyrolactone is a known compound and it is availa ble on the market. It can be prepared, for instance, according

to the method described by J.E.Livak et al. J.Am.Chem. Soc. 67; 2218, (1945).

A further object of the present invention are the pharmaceutical compositions containing as active ingredient a compound of the general formula (I), together with pharmaceutically acceptable excipients.

The finished pharmaceutical forms suitable for the oral administration can be capsules, tablets, pellets, granulated product.

The following examples are intended for illustration of the present invention; they are not intended to limit the invention in any way.

Example 1

Preparation of α-n.butylamino-γ-butyrolactone

Method A)

1 mol of α-amino-γ-butyrolactone is mixed with more than 2 mols of pyridine. To the obtained mixture, cooled to 0 ± 5°C there is added 1-1.25 mol of n.butyl-chloride.

The mixture is allowee to stand at about 0°C for a time sufficient to complete the reaction. Thereafter, pyridine hydrochloride is filtered off and the remaining mixture is heated at room temperature. The mixture is concentrated by evaporation and thereafter it is cooled on ice; α-butylamino-γ-butyrolactone crystallizes. Yield 85% on the theoretic. m.p. 62-63 °C.

Method B)

1 mol of α-bromo-γ-butyrolactone is mixed with more than 2 mols of pyridine and the obtained mixture, cooled to 0±5°C, is added with 1-1.25 mol of n.butylamine.

The reaction mixture is allowed to stand for a time sufficient to complete the reaction; thereafter, pyridine hydrobromide is filtered off and the liquid is heated at room temperature. After con

centration by evaporation, the solution is allowed to crystallize to afford α-n.butylamino-γ-butyrolactone. m.p. 62-63°C.

Example 2

Preparation of α-pentylamino-γ-butyrolactone

Method A)

1 mol of α-amino-γ-butyrolactone is mixed with more than 2 mols of pyridine.

The mixture is cooled to 0 ± 5°C and is added with 1-1.25 mol of n.pentylchloride. The mixture is allowed to stand at 0°C for a time sufficient to complete the reaction. After having filtered the pyridine hydrochloride, the solution is heated at room temperature and is concentrated by evaporation. The concentrated solution is cooled on ice and allowed to crystallize to afford α-n.pentylamino-γ-butyrolactone. m.p. 64-65 °C.

Method B)

By working under the same conditions described for Method A), but replacing α-amino-γ-butyrolactone by 1 mol of α-bromo-γ-butyrolactone and allowing it to react with n.pentylamine, the same compound is obtained as by Method A), that is α-n.pentylamine-γ-butyrolactone.

Example 3

Wistar rats have been selected in order to test the effectiveness of the compounds of the invention to suppress the alcohol desire. After having administered by i.p. injection an anhestetic dose of 4.5 g/kg of ethanol (25% solution), the sensitiveness of rats to the soporific effect of ethanol was tested. The beginning and the duration of the sleep period, indicated by the disappearance and recurrence of the "righting" reflex were recorded.

Animals showing a shorter sleep period were placed into single cages and kept under a cyclic period (12 hours) of light and dark.

The same processes were applied to rats showing a longer sleep period.

An ethanol (15%) solution and water were administered in graduated bottles. The position of the bottles was irregularly changed to avoid the use of a certain position and to make sure that rats ingest from beginning both the available liquids. Ingestion of liquids has been recorded each day at the same hour.

On ly the animals showing the shorter sleep period began to drink ethanol solution. These animals, in fact, ingested 4.3±0.85 g/kg/day of ethanol after two weeks, whereas the animals showing the longer sleep period ingested only 0.75±0.13 g/kg/day.

After 1 month exposure to alcohol and after having recorded sample values of alcohol ingestion, the animals showing the shorter sleep period were divided randomly in two groups.

During three subsequent days, a group was given with α-n.butylamino-γ-butyrolactone at the level of 200 mg/kg i.p., together with a pharmaceutically suitable vehicle, twice a day.

Saline solution was injected to the group of controls.

The verification of the ingestion of liquid and the verification of body weight was continued for further 5 days after the end of the injection period.

The following table shows the average consumption of ethanol expressed as absolute amount of ethanol before, during and after the injection with α-n.butylamino-γ-butyrolactone.

Significant differences as to the ethanol consumption were not observed in the group of controls; on the contrary, in the group of the animals treated with α-n.butylamino-γ-butyrolactone, and indicated as test group, a complete suppression of the ingestion of ethanol was observed. The consumption of alcohol remained at low levels during 5 days after the injection. Concomitantly with

the suppression of the voluntary ingestion of ethanol, neither significant reduction of the total swallowing of liquid was observed nor of the body weight.

The data reported in the following table show that the most part of the liquid ingested by rats daily, namely 15%, was ethanol solution, corresponding to 4-5 g ethanol/kg/day.

I.p. injection of PTT-1 at the dose of 200 mg/kg/day, for three days in succession, resulted in about 80% reduction of the ethanol ingestion during the treatment, but did not reduce the total ingestion of liquid. Ethanol ingestion was kept remarkably reduced up to the fifth day after the interruption of the treatment.

It has been observed that up to a $10^{-3}$ M concentration, PTT-1 did not inhibit eiether alcoholic dehydrogenase or the aldehydo-dehydrogenase in rat homogenized liver, or the dopamine-β-hydroxylase in rat homogenized medulloid parts of adrenal gland or of rat homogenized hypothalamus.

T A B L E

Effectiveness of PTT-1 in suppressing alcohol ingestion

Total fluid ingested (ml)

| | Days | Controls | Treated animals |
|---|---|---|---|
| | 1 | 32.0 | 30.0 |
| | 3 | 32.0 | 30.0 |
| treatment with PTT-1 | 5 | 36.0 | 35.5 |
| treatment with PTT-1 | 8 | 35.0 | 34.5 |
| | 11 | 35.2 | 32.0 |

Ethanol consumption (g/kg)

| | Days | Controls | Treated animals |
|---|---|---|---|
| | 1 | 4.3 | 4.0 |
| | 3 | 4.3 | 4.2 |
| treatment with PTT-1 | 5 | 4.25 | 4.25 |
| treatment with PTT-1 | 8 | 4.4 | 0.4 |
| | 11 | 4.4 | 1.1 |
| | 16 | 4.2 | 1.3 |

Example 4

12 alcohol -depending voluntary humans were given 2 g/day of the compound PTT-1, for 2 days. Each tablet administered contained 0.5 g of PTT-1; tablets were administered 4 times a day. At the end of the treatment, all the subjects showed a little, or even no desire of intoxicating drinks. Although patients ingested intoxicating drink after the administration of PTT-1, no potentially very dangerous reaction was observed, which appeared when alcohol was ingested after the administration of disulfiram. Furthermore, patients showed a little or even no desire to drink again intoxicating drinks for about 30 days after the treatement with PTT-1.

C L A I M S

1. γ-Butyrolactone derivatives able to inhibit ingestion of alcohol or intoxicating drinks, comprised in the general formula (I):

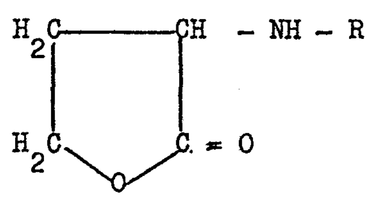

(wherein R is a linear or branched alkyl radical containing from 3 to 5 Carbon atoms) and their pharmaceutically acceptable salts.

2. α-n.butylamino-γ-butyrolactone.

3. The method for the preparation of γ-butyrolactone derivatives of claim 1, characterized in that an alkylhalide is allowed to react with an α-amino-γ-butyrolactone, at a low temperature and in the presence of a tertiary organic base.

4. The method for the preparation of γ-butyrolactone derivatives of claim 1, characterized in that an α-halo-γ-butyrolactone is allowed to react with the selected alkylamine, at low temperature and in the presence of a tertiary organic base.

5. Pharmaceutical compositions for inhibiting alcohol or intoxicating drinks ingestion, characterized in that they contain a compound of the general formula (I)

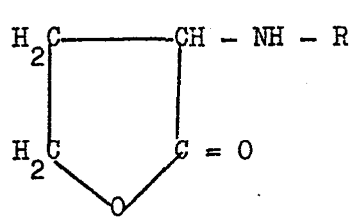

(wherein R is a linear or branched alkyl radical containing from 3 to 5 Carbon atoms) or a pharmaceutically acceptable salt thereof as active ingredient, together with a suitable pharmaceutically acceptable vehicle.

6. Pharmaceutical compositions for inhibiting alcohol or intoxicating drinks ingestion, characterized in that they contain as active ingredient the α-n°butylamino-γ-butyrolactone, together with a suitable pharmaceutically acceptable vehicle.

0144812

European Patent Office

# EUROPEAN SEARCH REPORT

Application number

EP 84 11 3636

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| A | GB-A-2 028 653 (GRISSMAN CHEM.)<br>* The whole document *<br>--- | 1-6 | C 07 D 307/32<br>A 61 K 31/365 |
| A | JOURNAL OF HETEROCYCLIC CHEMISTRY, Vol. 9, No. 5, October 1972, pages 979-984, PROVO, UTAH, (US). B. CAVALLERI et al.: "Synthesis of 1-Alkyl-2-nitroimidazole-5-carboxaldehydes".<br>* Page 983, left-hand column, last paragraph, right-hand column, paragraph 1 *<br>----- | 1-4 | |
| | | | TECHNICAL FIELDS SEARCHED (Int. Cl 4) |
| | | | C 07 D 307/00 |

The present search report has been drawn up for all claims

| Place of search | Date of completion of the search | Examiner |
|---|---|---|
| THE HAGUE | 12-02-1985 | ALLARD M.S. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document